# EUROPÄISCHE PATENTANMELDUNG

(11) **EP 2 272 823 A1**
(43) Veröffentlichungstag der Anmeldung: **12.01.2011**
(21) Anmeldenummer: 10182100.7
(22) Anmeldetag: 23.06.2005
(51) Int. Cl.: C07C 245/16

(54) **Verfahren zur Herstellung von gasförmigen Diazoalkanen**

(30) Priorität: 23.06.2004 DE 102004030371
(62) Teilanmeldung aus: 05769637.9
(71) Anmelder: Dynamit Nobel GmbH Explosivstoff- und Systemtechnik, 51377 Leverkusen (DE)
(72) Erfinder: Haase, Jürgen, 45731 Waltrop (DE)
(74) Vertreter: Hirsch & Associés

(57) **Zusammenfassung**

Beschrieben wird ein Verfahren zur Herstellung von Diazoalkanen, bei dem ein Diazoalkanprecursor in einem ersten Lösungsmittel gelöst wird, eine Base in einem zweiten Lösungsmittel gelöst wird, die Stoffe in einem Reaktionsbehälter unter Bildung des Diazoalkans reagieren und das erhaltene Diazoalkan unter reduziertem Druck abgeführt wird. Das Verfahren eignet sich besonders zur Herstellung von Diazomethan.

## Beschreibung

Die Erfindung betrifft allgemein ein Verfahren zur Herstellung von gasförmigen Diazoalkanen und besonders ein Verfahren zur Herstellung von Diazomethan.

Diazoalkane spielen als Zwischenprodukte / Reaktanten in der organischen Synthese eine große Rolle, insbesondere bei Veretherungsreaktionen von NH-, OH- oder aciden Bindungen, bei Heterocyclensynthesen, bei Additionen an Doppelbindungssysteme oder bei Einschubreaktionen unter milden Bedingungen.

Ein in der WO 01/47869 beschriebenes Verfahren zur Herstellung von Diazomethan enthält die Schritte:
- Zugabe eines Diazomethanprecursors gelöst in einem ersten Lösungsmittel und
- Zugabe einer Base gelöst in einem zweiten Lösungsmittel (bevorzugt Wasser) in einen Reaktionsbehälter, in dem diese Stoffe unter Bildung von Diazomethan reagieren. Das erhaltene Diazomethan wird mit Hilfe eines verdünnenden Gases abgeführt, wobei dieser Gasstrom im wesentlichen frei ist von Lösungsmitteln, bzw. Lösungsmitteldämpfen.

Nachteil dieses Verfahrens ist, dass
- durch den aus Sicherheitsgründen notwendigen hohen Inertgasstrom deutliche Mengen Wasser (das bei diesem Verfahren bevorzugte zweite Lösungsmittel) aus dem Reaktionsbehälter abgestrippt werden, die über großflächige Kühler und/oder Trockeneinrichtengen dem Gasstrom entzogen werden müssen, wenn sie bei Umsetzungsreaktion stören. Hierdurch fallen hohe Investitionskosten bei einem Anlagenbau an.
- Aus Sicherheitsgründen muss der Diazomethangehalt im Gasstrom unter ca. 15 % gehalten werden. Dies erfordert eine aufwändige und damit teure Online-Messung (z.B. mittels Infrarotspektroskopie) mit dazugehörender Inertgas-Regelungstechnik.
- Diazoalkane sind sehr giftig und krebserregend. Bei Verwendung eines verdünnenden Inertgases bei der Herstellung von Diazoalkanen wird zwangsläufig mit leichtem Überdruck gearbeitet. Bei Leckagen in der Anlage treten daher sofort Diazoalkangase in die Umgebungsluft aus und können dort Menschen gefährden.

Aufgabe der Erfindung ist es, die Nachteile des Standes der Technik zu überwinden und ein Verfahren zur Herstellung von gasförmigen Diazoalkanen und im besonderen zur Herstellung von Diazomethan bereitzustellen, welches ohne die Hilfe eines verdünnenden Gases auskommt.

Gelöst wird die Aufgabe durch ein Verfahren zur Herstellung von gasförmigen Diazoalkanen, bei dem ein Diazoalkanprecursor in einem ersten Lösungsmittelund eine Base in einem zweiten Lösungsmittel gelöst wird, die Stoffe in einem Reaktionsbehälter unter Bildung des Diazoalkans reagieren und das erhaltene Diazoalkan unter gegenüber Normaldruck reduziertem Druck abgeführt wird.

Überraschend wurde gefunden, dass Diazomethan im einem unter Normaldruck liegenden Druckbereich ähnliche Explosionsgrenzen wie bei der Verdünnung mit Inertgas aufweist und nicht durch Zündfunken zur Explosion gebracht werden kann.

Als Diazoalkanprecursor können z.B. folgende Verbindungen eingesetzt werden: N-Alkyl-N-nitroso-Verbindungen, wie N-Alkyl-N-nitrosocarbonsäureamide (z.B. N-Alkyl-N-nitrosoharnstoff), N-Alkyl-N-nitrosourethane, N-Alkyl-N-nitrosoaryl-sulfonsäureamide (z.B. N-Alkyl-N-nitrosotoluol-sulfonsäureamide) oder N-Alkyl-N-nitrosoaminoketone.

Als bevorzugtes Diazoalkan kann mit dem erfindungsgemäßen Verfahren Diazomethan hergestellt werden. Als Diazomethanprecursor können alle dafür bekannten und z.B. in der WO 01/47869 beschriebenen Verbindungen eingesetzt werden, insbesondere N-Methyl-N-nitroso-Verbindungen, wie N-Methyl-N-nitrosocarbonsäureamide (z.B. N-Methyl-N-nitrosoharnstoff), N-Methyl-N-nitrosourethane, N-Methyl-N-nitrosoaryl-sulfonsäureamide (z.B. N-Methyl-N-nitrosotoluol-sulfonsäureamid) oder N-Methyl-N-nitrosoaminoketone.

Als Base können anorganische Basen wie z.B. Lithium-, Natrium-, Kalium-, Rubidium-, Cesium- oder Bariumhydroxid eingesetzt werden. Als Base können ebenso organische Basen, wie z.B. N-Heterocyclen eingesetzt werden. Aus Kostengründen bevorzugt eingesetzt werden die Alkalihydroxide Natrium- oder Kaliumhydroxid.

Das erste und das zweite Lösungsmittel können gleich oder verschieden sein.

Als erstes Lösungsmittel, in dem der Diazoalkanprecursor gelöst wird, werden bevorzugt (z.B. aus der WO 01/47869) bekannte Lösungsmittel eingesetzt, die einen hohen Siedepunkt, einen niedrigen Dampfdruck und eine hohe Wasserlöslichkeit aufweisen. Ferner ist das erste Lösungsmittel bevorzugt nicht reaktiv gegenüber Diazoalkanen. Als erstes Lösungsmittel werden besonders bevorzugt eingesetzt Arylalkyl-Ether, Glykolether, Dimethylformamid (DMF) Dimethylsulfoxid (DMSO) oder ionische Lösungsmittel, beispielsweise 1,3-Dimethyl-imidazolinium Dimethylphospat. Ganz besonders bevorzugt werden DMSO und/oder Di(ethylenglykol)ether verwendet. Es können auch Gemische der genannten Lösungsmittel eingesetzt werden.

Das zweite Lösungsmittel, in dem die Base gelöst wird, kann ausgewählt werden aus einem Lösungsmittel, das als erstes Lösungsmittel eingesetzt werden kann. Dabei können das erste und das zweite Lösungsmittel identisch sein, müssen es aber nicht. Bevorzugt ist das zweite Lösungsmittel Wasser oder enthält Wasser.

Bevorzugt wird die Base (kann aus mehreren Stoffen bestehen) in einer Menge von 1 bis 3 Equivalenten relativ zum Diazoalkanprecursor eingesetzt. Besonders bevorzugt ist eine Menge von 1,1 bis 2 Equivalenten, so dass die Base im Überschuss vorliegt.

Der Diazoalkanprecursor und die Base können gleichzeitig oder nacheinander zugegeben und miteinander vermischt werden.

Unter reduziertem Druck wird ein gegenüber dem Normaldruck reduzierter Druckbereich verstanden. Bevorzugt beträgt der reduzierte Druckbereich 0 bis 800 mbar, besonders bevorzugt 100 bis 300 mbar. Das Abführen des Diazoalkans unter reduziertem Druck kann unter Zuhilfenahme eines Inertgases durchgeführt werden. Bevorzugt wird das Diazoalkan jedoch unter reduziertem Druck ohne Zuhilfenahme eines Inertgases abgeführt.

Die Reaktion wird bevorzugt bei Temperaturen von 10 bis 80°C, besonders bevorzugt von 20 bis 50°C durchgeführt.

Die Reaktion der Diazoalkangenerierung kann kontinuierlich oder batchförmig durchgeführt werden.

Das erfindungsgemäße Verfahren kann beispielhaft wie folgt durchgeführt werden (ohne dass mit dieser Beschreibung irgendwelche Einschränkungen vorgenommen werden):
In einem Diazomethan - Generierungsreaktor wird eine ca. 25 %-ige Lösung von N-Methyl-N-nitroso-toluolsulfonsäureamid in DMSO bei 20 bis 70°C gleichzeitig oder nacheinander mit einer 20 bis 40 %-igen wässrigen KOH Lösung vermischt.
Das sich spontan bildende Diazomethan wird mittels einem Unterdruck von 0 bis 800 mbar in einen Umsetzungsreaktor oder Reaktionswäscher gezogen, wo es nach gewünschter Weise abreagiert.

Das erfindungsgemäße Verfahren hat gegenüber bisher bekannten Verfahren den Vorteil, dass die Menge des Inertgases erheblich reduziert werden kann, bzw. auf das Inertgas ganz verzichtet werden kann. Allenfalls unbedeutende Lösungsmitteldampfmengen (wie z.B. Wasserdampf) werden aus dem Diazoalkanangenerator abgestrippt, die gegebenenfalls mit kleinflächigen Kondensatoren oder Trockeneinrichtungen aus dem Diazoalkan entfernt werden können. Durch die Vakuumfahrweise wird bei einer auftretenden Leckage eine Gefährdung des Bedienungspersonals durch die sehr giftigen und krebserregenden Diazoalkangase ausgeschlossen. Weiterhin kann die aus sicherheitstechnischen Gründen notwendige Diazoalkan-Konzentrationsabsenkung allein durch eine preisgünstige Druckmessung realisiert werden. Sicherheitsuntersuchungen an DAM im Vakuum haben gezeigt, dass bei einem Partialdruck > 150 mbar DAM mittels eines Zündfunkens zur Explosion gebracht werden kann. Diese "Konzentration" an DAM sollte daher in einer technischen Anlage möglichst nicht überschritten werden. Ergänzt man diesen Partialdruck mit Inertgas auf Normaldruck, so entspricht diese Grenze einer DAM Konzentration von ca. 15 Vol%, ein Wert, der als Explosionsgrenze bei Normaldruck für DAM angegeben wird.

Der Partialdruck des entstehenden Diazoalkans, beispielsweise des Diazomethans (DAM), wird erfindungsgemäß kontinuierlich mittels Infrarotspektroskopie gemessen.

Die nachfolgenden Beispiele sollen die Erfindung erläutern, ohne sie einzuschränken:

### Beispiel 1-3:

In einem 1,5 I Doppelmantelglasreaktor wird 531 g wässrige 30 %-ige KOH - Lösung vorgelegt und jeweils auf 40; 55 bzw. 70°C erwärmt. Durch kontrollierte Zutropfung von 405,8 g 26,4 %-iger N-Methyl-N-nitroso-p-toluolsulfonsäureamid (MNTSA) - Lösung in DMSO in diese KOH - Lösung wird Diazomethan (DAM) erzeugt und gasförmig mittels Vakuum bei 500 mbar über eine Fritte in einen Glasreaktor gesaugt. Die Zudosierung erfolgt so, dass die Diazomethan-Konzentration in der Gasphase einen Partialdruck von 120 mbar nicht überschreitet. Zur Bestimmung der DAM Menge und Ausbeute wird dieser Glasreaktor mit einer Säure z.B. mit einer Benzoesäure - Lösung in DMF beschickt, so dass das über die Fritte eingesaugte DAM sofort zu Benzoesäuremethylester abreagiert und dessen Gehalt analytisch bestimmt und als Equivalent für DAM genutzt werden kann. Die folgende Tabelle gibt die Resultate wieder:

| Beispiel Nr. | Reaktionstemperatur [°C] | Ausbeute DAM [%] |
|---|---|---|
| 1 | 40 | 32,8 |
| 2 | 55 | 28,4 |
| 3 | 70 | 23,3 |

### Beispiel 4-6:

Der Versuchsaufbau und Ausbeutebestimmung entspricht dem der Versuche 1-3. In die vorgelegten 561 g 30 %-iger KOH - Lösung wird ohne Unterbrechung 259,7 g 25,9 %-iger N-Methyl-N-nitroso-p-toluolsulfonsäureamid - Lösung in DMSO bei 40°C zugetropft. Bei dieser Vorgehensweise wird das Vakuum durch angepasste Zuleitung eines Argonstromes von 800 über 500 auf 320 mbar schrittweise geändert. Die Zudosierung der Nitrosomethyl-Komponente erfolgt so, dass ein DAM Partialdruck von 150 mbar nicht überschritten wird.

| Beispiel Nr. | Reaktionsdruck [mbar] | Ausbeute DAM [%] |
|---|---|---|
| 4 | 800 | 41 ,0 |
| 5 | 500 | 46,4 |
| 6 | 320 | 47,6 |

### Beispiel 7-9:

Die Versuchsanordnung entspricht der der Versuche 1-3.

Die 30 %-ige KOH - Lösung wird parallel zur 25,9 %-igen N-Methyl-N-nitroso-p-toluolsulfonsäureamid - Lösung in DMSO bei 40°C in den Reaktor bei 500 mbar unter Einhaltung des DAM Partialdruckes von 150 mbar eindosiert. In den Versuchen wird das Verhältnis von KOH zur Nitrosomethyl-Komponente von 1,1 bis 1,5 verändert.

| Beispiel Nr. | Dosier-geschwindigkeit KOH Lösung [ml/min] | Dosier-geschwindigkeit MNTSA Lösung [ml/min] | Mol-Verhältnis KOH / MNTSA | Ausbeute DAM [%] |
|---|---|---|---|---|
| 7 | 0,330 | 1,466 | 1,1 | 47,2 |
| 8 | 0,375 | 1,466 | 1,25 | 47,6 |
| 9 | 0,450 | 1,466 | 1,5 | 60,9 |

### Beispiel 10:

In einem Langzeitversuch wird über 4 Stunden wie in den Versuchen 7-9 vorgegangen, indem eine 22,5 %-ige MNTSA Lösung gleichzeitig zu einer 30%-igen KOH Lösung zudosiert werden.

| Beispiel Nr. | Dosier-geschwindigkeit KOH Lösung [ml/min] | Dosier-geschwindigkeit MNTSA Lösung [ml/min] | Mol-Verhältnis KOH/ MNTSA | Ausbeute DAM [%] |
|---|---|---|---|---|
| 10 | 0,450 | 1,735 | 1,5 | 68,3 |

### Beispiel 11:

Abweichend zu dem Versuch 7 wurde MNTSA in dem ionischen Lösemittel 1,3-Dimethyl-imidazolinium Dimethylphospat (ECOENG 1111 P der Fa. Solvent Innovation GmbH) als 8,0 %-ige Lösung parallel mit einer 30%-igen KOH Lösung eindosiert.

| Beispiel Nr. | Dosier-geschwindigkeit KOH Lösung [ml/min] | Dosier-geschwindigkeit MNTSA Lösung [ml/min] | Mol-Verhältnis KOH / MNTSA | Ausbeute DAM [%] |
|---|---|---|---|---|
| 11 | 0,450 | 4,381 | 1,5 | 11,3 |

## Patentansprüche

1. Verfahren zur Herstellung von gasförmigem Diazoalkan, **dadurch gekennzeichnet, dass** ein Diazoalkanprecursor in einem ersten Lösungsmittel und eine Base in einem zweiten Lösungsmittel gelöst wird und die Stoffe in einem Reaktionsbehälter unter Bildung von Diazoalkan reagieren, wobei das erhaltene gasförmige Diazoalkan unter reduziertem Druck abgeführt wird.

2. Verfahren nach Anspruch 1, **dadurch gekennzeichnet, dass** als Diazoalkanprecursor N-Alkyl-N-nitroso-Verbindungen, wie N-Alkyl-N-nitrosocarbonsäureamide (z.B. N-Alkyl-N-nitrosoharnstoff), N-Alkyl-N-nitrosourethane, N-Alkyl-N-nitrosoaryl-sulfonsäureamide (z.B. N-Alkyl-N-nitrosotoluol-sulfonsäureamide) oder N-Alkyl-N-nitrosoaminoketone eingesetzt werden.

3. Verfahren nach Anspruch 1 oder 2 zur Herstellung von Diazomethan.

4. Verfahren nach Anspruch 3, **dadurch gekennzeichnet, dass** als Diazomethanprecursor N-Methyl-N-nitroso-Verbindungen, wie N-Methyl-N-nitrosocarbonsäureamide (z.B. N-Methyl-N-nitrosoharnstoff), N-Methyl-N-nitrosourethane, N-Methyl-N-nitrosoaryl-sulfonsäureamide (z.B. N-Methyl-N-nitrosotoluol-sulfonsäureamid) oder N-Methyl-N-nitrosoaminoketone eingesetzt werden.

5. Verfahren nach einem der Ansprüche 1 bis 4, **dadurch gekennzeichnet, dass** als Base anorganische oder organische Basen eingesetzt werden.

6. Verfahren nach Anspruch 5, **dadurch gekennzeichnet, dass** die anorganische Base ausgewählt ist aus einem oder mehreren der Stoffe Lithium-, Natrium-, Kalium-, Rubidium-, Cesium- und Bariumhydroxid.

7. Verfahren nach Anspruch 5, **dadurch gekennzeichnet, dass** als organische Base Stickstoffheterocyclen (z.B. N-Methylmorpholin) eingesetzt werden.

8. Verfahren nach einem der Ansprüche 1 bis 7, **dadurch gekennzeichnet, dass** als erstes Lösungsmittel ein Arylalkyl-Ether, Glykolether (z.B. Di(ethylenglykol)ether), Dimethylformamid (DMF), Dimethylsulfoxid (DMSO), ionische Lösungsmittel, beispielsweise 1,3-Dimethyl-imidazolinium Dimethylphospat oder Gemische dieser Stoffe eingesetzt wird.

9. Verfahren nach einem der Ansprüche 1 bis 8, **dadurch gekennzeichnet, dass** als zweites Lösungsmittel ein Lösungsmittel aus der Gruppe der ersten Lösungsmittel ausgewählt wird, wobei das erste und das zweite Lösungsmittel identisch sein können, aber nicht müssen.

10. Verfahren nach einem der Ansprüche 1 bis 8, **dadurch gekennzeichnet, dass** als zweites Lösungsmittel Wasser ausgewählt ist oder dass das zweite Lösungsmittel Wasser enthält.

11. Verfahren nach einem der Ansprüche 1 bis 10, **dadurch gekennzeichnet, dass** die Base in einer Menge von 1 bis 3 Equivalenten relativ zum Diazoalkanprecursor eingesetzt wird.

12. Verfahren nach einem der Ansprüche 1 bis 11, **dadurch gekennzeichnet, dass** der Diazoalkanprecursor und die Base gleichzeitig zugegeben und miteinander vermischt werden.

13. Verfahren nach einem der Ansprüche 1 bis 11, **dadurch gekennzeichnet, dass** der Diazoalkanprecursor und die Base nacheinander zugegeben und miteinander vermischt werden.

14. Verfahren nach einem der Ansprüche 1 bis 13, **dadurch gekennzeichnet, dass** das erhaltene gasförmige Diazoalkan bei 0 bis 800 mbar abgeführt wird.

15. Verfahren nach Anspruch 14, **dadurch gekennzeichnet, dass** das erhaltene gasförmige Diazoalkan bei 100 bis 300 mbar abgeführt wird.

16. Verfahren nach einem der Ansprüche 1 bis 13, **dadurch gekennzeichnet, dass** die Reaktion bei Temperaturen von 10 bis 80°C erfolgt.

17. Verfahren nach Anspruch 16, **dadurch gekennzeichnet, dass** die Reaktion bei Temperaturen von 20 bis 50°C erfolgt.
